# FASCICULE DE BREVET EUROPEEN

(11) **EP 0 454 524 B1**
(45) Date de publication et mention de la délivrance du brevet: **20.07.1994**
(21) Numéro de dépôt: 91400940.2
(22) Date de dépôt: 08.04.1991
(51) Int. Cl.: A61F 2/60

(54) **Prothèse de jambe à ossature monobloc et son procédé de fabrication**
Beinprothese mit einstückigem Gerüst und Verfahren zu ihrer Herstellung
Leg prosthesis with one-piece frame and process for manufacturing the same

(30) Priorité: 23.04.1990 FR 9005126
(43) Date de publication de la demande: 30.10.1991
(73) Titulaire: ETABLISSEMENTS PROTEOR, 21100 Dijon (FR)
(72) Inventeur: Palfray, Michel, F-21250 Seurre (FR)
(74) Mandataire: Jolly, Jean-Pierre

(56) Documents cités:
- WO-A-89/09036
- FR-A- 690 657
- FR-A- 1 162 321
- FR-A- 2 638 087
- GB-A- 2 202 448
- US-A- 2 379 538
- US-A- 3 909 855
- US-A- 4 911 724

## Description

La présente invention concerne une prothèse à ossature monobloc pour amputation de la jambe. L'invention concerne également un procédé de fabrication d'une telle prothèse.

Les prothèses usuelles pour amputation de la jambe comprennent généralement trois éléments principaux :
- une partie formant fourreau, dite emboîture, dans laquelle vient se loger le moignon de la personne amputée et qui peut être rendue solidaire de ce moignon;
- un pied prothétique;
- et une partie de jambe réunissant l'emboîture et le pied prothétique.

Ces éléments sont réalisés de façon séparée et sont ensuite assemblés pour former la prothèse, (voir, par exemple FR-A-1 162 321 et WO-A-89/09036). Ils peuvent être constitués de différents matériaux, mais la prothèse résultante est en général lourde, en particulier en raison de la structure du pied prothétique, et celui-ci a souvent une médiocre résistance mécanique.

De ce fait, ces prothèses conviennent mal aux personnes âgées, qui, d'une part, se déplacent très peu et n'ont donc pas besoin d'une prothèse complexe, et qui, d'autre part, se fatiguent très rapidement, si la prothèse est trop lourde.

US-A-3 909 855 décrit une prothèse monobloc du type "au-dessous du genou", dans laquelle une partie de jambe et une partie de pied forment un ensemble monobloc moulé. La partie de pied est cependant rigide et ne permet aucune souplesse par rapport à la partie de jambe.

La présente invention vise à remédier à ces inconvénients en proposant une prothèse pour amputation de la jambe qui soit beaucoup plus légère et d'une structure plus simple que celles des techniques classiques et qui convienne ainsi particulièrement bien à une utilisation par des personnes âgées.

L'invention a également pour but de proposer une prothèse de ce type qui puisse être réalisée sous forme monobloc par un procédé facile à mettre en oeuvre.

Un autre but de l'invention est de proposer une prothèse du type ci-dessus, dont le pied prothétique présente une grande souplesse à la marche, en lui offrant un effet de restitution d'énergie. L'invention a enfin pour but de proposer une telle prothèse qui soit d'un coût de fabrication réduit.

A cet effet, l'invention a pour objet une prothèse pour amputé de la jambe comprenant une ossature thermoformée en une matière thermoplastique ou en un matériau stratifié, gainée d'une matière de revêtement et comprenant une emboîture, une partie de jambe et un pied prothétique attenants deux à deux, l'ossature de la partie de jambe et du pied prothétique constituant un ensemble monobloc, venu d'une unique opération de moulage, cette prothèse étant caractérisée et en ce que le pied prothétique comprend, au niveau de la semelle, une découpe ayant servi à l'extraction du noyau de moulage et, au niveau du talon, au-dessus de la semelle, une découpe en forme de coin se prêtant à une flexion de l'arrière de la semelle par rapport à la partie de jambe.

De préférence, l'emboîture sera également venue de moulage avec la partie de jambe et le pied prothétique, mais elle pourra aussi être rapportée sur celles-ci et comporter par exemple une partie saillante à section en U à sa partie inférieure, apte à coiffer une partie saillante de profil complémentaire ménagée à la partie supérieure de la partie de jambe et destinée à être fixée sur celle-ci dans la position la plus appropriée à l'aide de vis et d'écrous.

L'invention a également pour objet un procédé de fabrication d'une telle prothèse, selon lequel on moule en une matière thermoplastique ou en un matériau stratifié une ossature monobloc de la partie de jambe et du pied prothétique autour d'un noyau approprié, caractérisé en ce qu'après moulage, on découpe la semelle du pied prothétique pour extraire le noyau, et en ce que l'on ménage dans l'ossature moulée, au niveau du talon du pied prothétique et au-dessus de la semelle, une découpe en forme de coin se prêtant à une flexion de l'arrière de la semelle par rapport à la partie de jambe.

Avantageusement, une semelle de renfort en un matériau apte à se déformer élastiquement, en composite de carbone et de verre ou tout autre matériau apte à la déformation élastique, par exemple, sera incorporée dans le pied prothétique. Cette semelle pourra être fixée au-dessous du noyau utilisé pour le moulage, préalablement à la réalisation de l'ossature monobloc de la prothèse.

Dans le but d'accroître les effets de restitution d'énergie à la prothèse, au cours de la marche, une lame ressort métallique pourra aussi être incorporée dans le pied prothétique au-dessus de la semelle en un matériau déformable élastiquement, en étant recourbée entre la partie avant et la partie arrière de la semelle.

Lorsque l'emboîture sera réalisée en même temps que la partie de jambe et le pied prothétique, également par moulage, une forme en plâtre reproduisant la forme du moignon sera fixée en position correspondante sur le noyau utilisé pour le moulage.

Avantageusement, à l'avant et à l'arrière de la partie de jambe seront ménagées des nervures, que l'on réalisera facilement, comme on l'exposera ci-après, en tendant obliquement un fil métallique entre la base de l'emboîture et la partie de jambe, à l'avant et à l'arrière de celle-ci.

D'autres caractéristiques et avantages de l'invention apparaîtront dans la description détaillée qui va suivre de diverses formes de réalisation, données à titre d'exemple non limitatif. Dans cette description, on se réfèrera aux dessins schématiques annexés, sur lesquels :
Les figures 1 et 2 sont des vues, respectivement en élévation latérale et de l'arrière d'une ossature monobloc de prothèse selon l'invention, avec le profil de la prothèse représenté en traits interrompus ;
La figure 3 est une vue à plus grande échelle de la partie inférieure de la figure 1 ;
La figure 4 est une vue suivant la flèche F₄ de la figure 3;
La figure 5 est une coupe avec arrachement partiel suivant la ligne V-V de la figure 3;
Les figures 6,7 et 8 sont des vues, respectivement, en élévation latérale, de face, et de dessus du noyau métallique utilisé pour effectuer le moulage de l'ossature de prothèse conforme à l'invention ;
La figure 9 est une vue analogue à la figure 8, dans le cas de la réalisation d'une ossature de prothèse avec l'emboîture attenante ;
La figure 10 illustre un exemple de thermoformage sous vide appliqué au noyau de la figure 9 ;
Les figures 11 et 12 sont des coupes transversales à plus grande échelle, illustrant deux phases du thermoformage de l'ossature au niveau de la ligne XI-XI de la figure 9 ;
La figure 13 est une coupe transversale, illustrant le thermoformage au niveau de la ligne XIII-XIII de la figure 9;
Les figures 14 et 15 sont des vues analogues respectivement, aux figures 3 et 5, d'une variante de l'ossature de prothèse conforme à l'invention dans laquelle une lame ressort recourbée est insérée dans le pied prothétique;
Les figures 16 et 17 sont des vues, respectivement en élévation latérale et de l'arrière, d'une autre variante de la prothèse selon l'invention, où l'emboîture est rapportée sur l'ossature thermoformée.

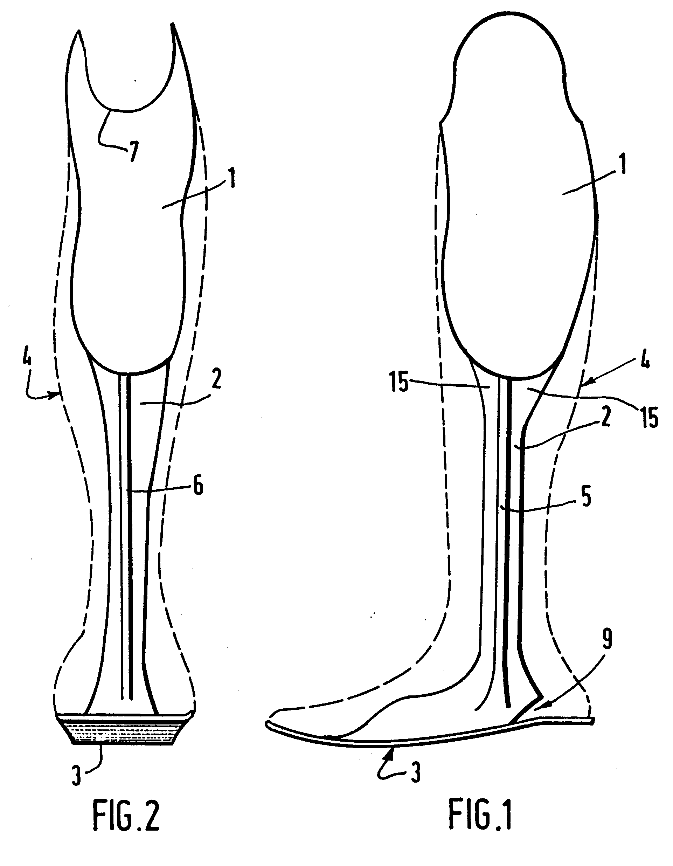

On se réfèrera d'abord aux figures 1 à 5, qui représentent une ossature monobloc de prothèse conforme à l'invention, avec une emboîture 1, destinée à recevoir le moignon de la personne amputée, une partie de jambe 2 attenante à l'emboîture 1 et venue de thermoformage avec celle-ci, et un pied prothétique 3, attenant à la partie de jambe 2 et venu de thermoformage avec celle-ci. Cette ossature est destinée à recevoir ensuite une garniture 4, par exemple en une matière cellulaire souple, imitant la forme du membre.

La partie de jambe 2 présente deux nervures latérales 5 et, à l'arrière, une nervure 6 formée par la soudure du matériau utilisé pour le thermoformage de l'ossature, une polyoléfine par exemple. L'emboîture 1 a été échancrée en 7 de façon usuelle après thermoformage.

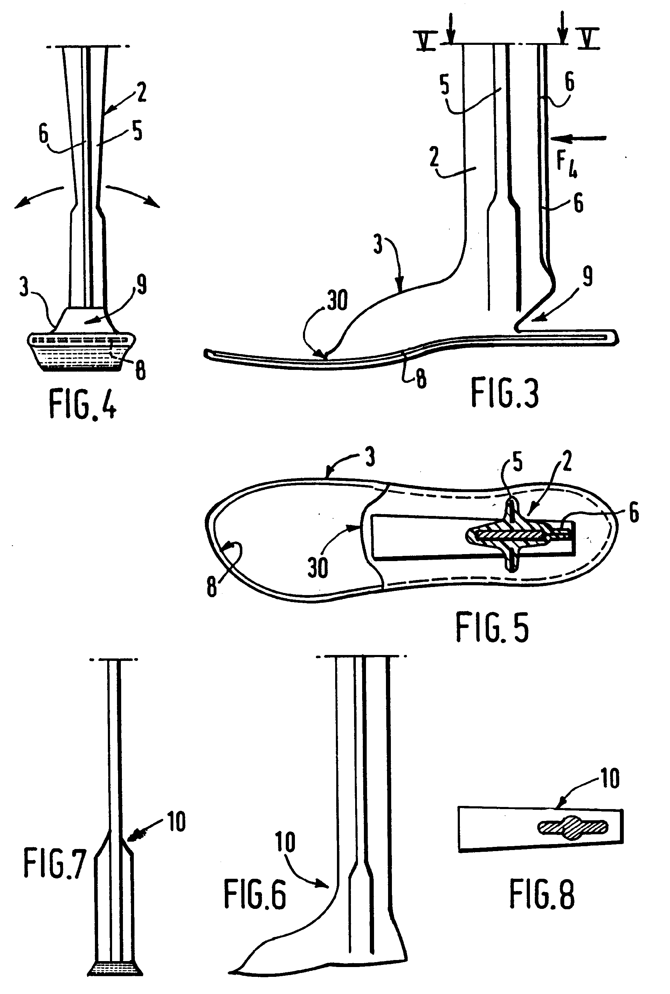

Comme on le voit plus clairement sur les figures 3, 4 et 5, le pied prothétique 3 comporte une semelle 8 de renforcement en composite de carbone ou tout autre matériau élastique, qui est enrobée, à l'avant et à l'arrière, du matériau de thermoformage et dont le but est de donner un effet de ressort au pied prothétique au cours de la marche de la personne amputée. On peut utiliser des semelles 8 différentes, suivant le poids et l'activité de la personne amputée, afin de moduler l'effet de ressort désiré. Pour conférer de la souplesse à la semelle thermoformée du pied prothétique et à la semelle 8, la partie supérieure avant du pied prothétique sera avantageusement découpée en 30, ainsi, éventuellement, que la partie de la semelle du pied prothétique disposée au-dessous de cette découpe 30. Dans le même but, une découpe 9 en forme de coin est ménagée au niveau du talon prothétique, entre la semelle et la partie de jambe, afin de conférer de la souplesse à la prothèse qui, à chaque pas de l'amputé, revient élastiquement à sa position de départ.

Le noyau métallique 10, servant à réaliser par moulage ou par thermoformage la prothèse conforme à l'invention, est représenté sur les figures 6,7 et 8.

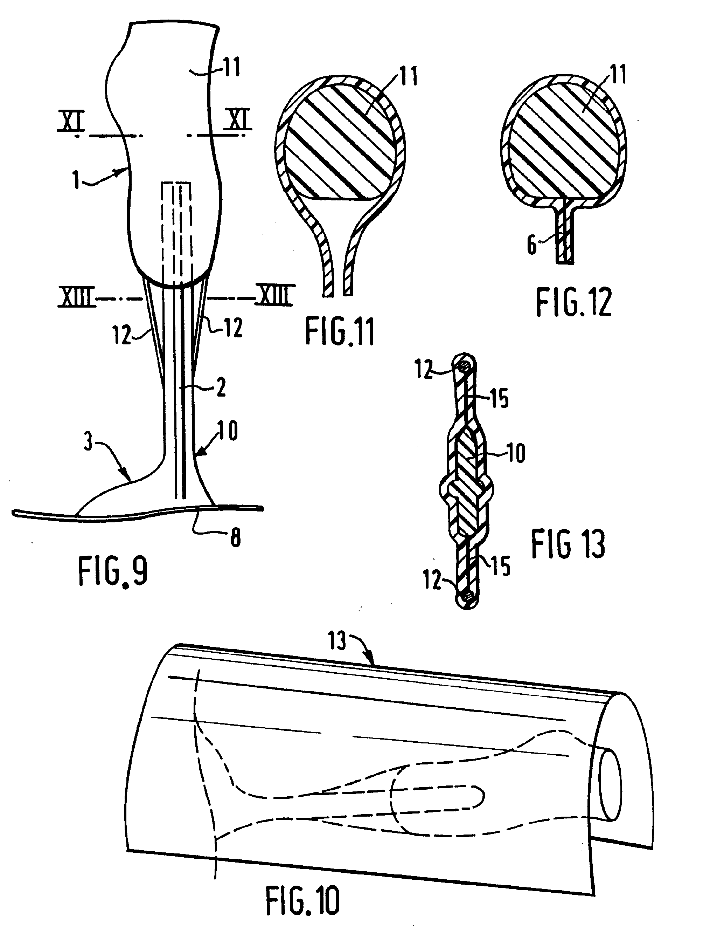

Avant de procéder au moulage, on fixe la semelle de renfort 8 sous la partie de base du noyau 10. Si l'on désire réaliser simultanément l'emboîture 1, on noie la tête du noyau 10 dans une reproduction en plâtre 11 du moignon de l'amputé 1. Afin de renforcer par des nervures 15, dirigées vers l'avant et vers l'arrière, la partie de l'ossature de la prothèse qui réunit la partie de jambe à l'emboîture, on tendra obliquement deux fils métalliques 12 entre la base du moulage en plâtre 11 du moignon et le noyau 10 (figure 9).

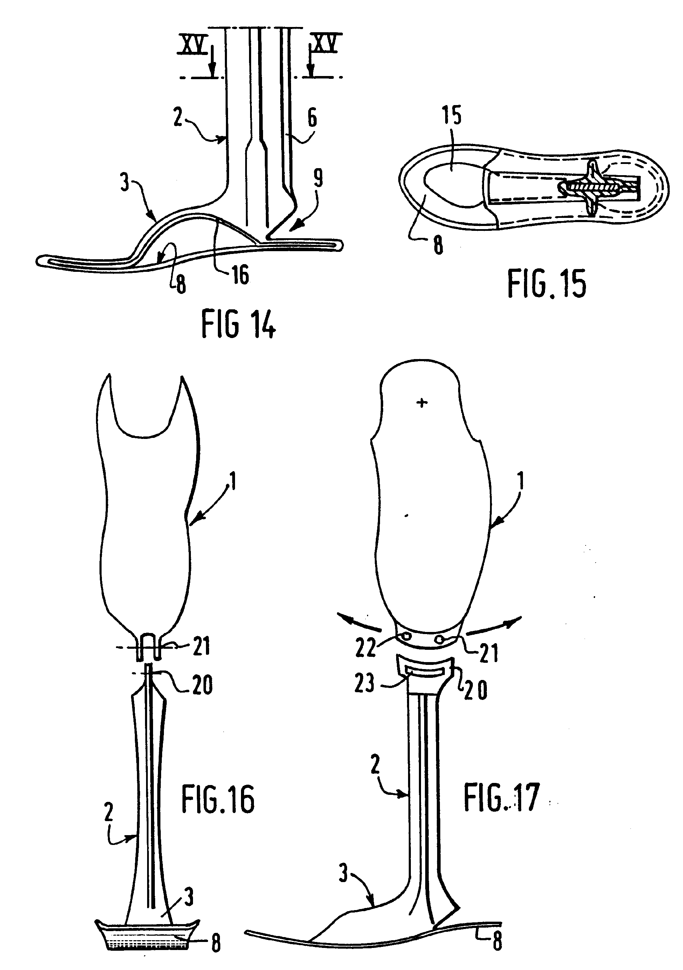

On procédera ensuite et par exemple de façon usuelle au thermoformage sous vide de l'ossature de prothèse, en enveloppant d'une feuille de polyéthylène 13 ou d'un autre matériau thermoplastique le noyau 10 ainsi équipé (figure 10), en chauffant la feuille 13 de manière qu'elle ramollisse (figure 11) et en la mettant sous dépression pour qu'elle s'applique étroitement contre le noyau 10, la semelle 8, le moulage 11 et les fils métalliques 12 (figures 12 et 13), en donnant ainsi naissance à la soudure 6, qui forme une nervure postérieure (figure 12), et à deux nervures latérales 15 de renfort (figure 13).

Après thermoformage de la prothèse, la semelle 8 de composite de carbone et sa gaine de polyéthylène sont découpées à la base du noyau 10, pour permettre l'extraction de celui-ci, et le moulage de plâtre 11 est détruit pour dégager l'emboîture 1, qui est ensuite découpée à la forme voulue.

On notera que la partie de jambe 2 peut avoir une section transversale de forme absolument quelconque.

Ainsi qu'il a été indiqué ci-dessus, on peut équiper d'une lame de ressort métallique le pied prothétique 3 de l'ossature de prothèse, afin d'avoir un effet accru de récupération d'énergie au cours de la marche de l'amputé.

C'est ce qui est représenté sur les figures 14 et 15, qui sont à rapprocher des figures 3 et 5. On voit qu'une lame de ressort 16 a été introduite dans le pied à travers l'ouverture ménagée pour l'extraction du noyau de thermoformage, et que la lame 16 est recourbée entre les extrémités avant et arrière du pied prothétique, contre lesquelles elle vient s'arc-bouter, en donnant ainsi à ce pied une souplesse accrue.

Enfin, les figures 16 et 17 illustrent une autre forme de réalisation de la prothèse conforme à l'invention. On voit, sur ces dessins, où les organes déjà décrits sont désignés par les mêmes chiffres de référence, que l'emboîture 10 est rapportée sur le corps monobloc thermoformé de la partie de jambe 2 et du pied prothétique 3.

Dans ce but, une partie saillante aplatie 20 est prévue à l'extrémité supérieure de la partie de jambe 2, tandis qu'une partie 21 à section en U, ménagée à la base de l'emboîture 1, peut coiffer cette partie 20. Des trous 22, percés dans la partie 21, et une lumière allongée 23, ménagée dans la partie 20, permettent d'assembler l'emboîture 1 et la partie de jambe 2 en une pluralité de positions, selon une courbe dont le centre et le rayon correspondent à ceux de l'articulation du genou, par exemple à l'aide de vis et d'écrous.

L'invention apporte donc une ossature monobloc de prothèse pour amputé de la jambe, qui peut être fabriquée par des moyens connus dans la technique, et qui n'en apporte pas moins un progrès technique appréciable pour l'amputé.

## Revendications

1. Prothèse pour amputé de la jambe comprenant une ossature thermoformée en une matière thermoplastique ou en un matériau stratifié, gainée d'une matière de revêtement et comprenant une emboîture (1), une partie de jambe (2) et un pied prothétique (3) attenants deux à deux, l'ossature de la partie de jambe (2) et du pied prothétique (3) constituant un ensemble monobloc, venu d'une unique opération de moulage, cette prothèse étant caractérisée en ce que le pied prothétique comprend, au niveau de la semelle, une découpe (30) ayant servi à l'extraction du noyau de moulage et, au niveau du talon, au-dessus de la semelle, une découpe (9) en forme de coin se prêtant à une flexion de l'arrière de la semelle par rapport à la partie de jambe.

2. Prothèse selon la revendication 1, caractérisée en ce que, dans le pied prothétique (3), est incorporée une semelle (8) en un matériau déformable élastiquement.

3. Prothèse selon la revendication 2, caractérisée en ce que, dans le pied prothétique 3, au-dessus de la semelle (8) en un matériau déformable élastiquement, est incorporée une lame ressort métallique (16), renflée en sa partie centrale et prenant appui par ses extrémités contre l'extrémité avant et l'extrémité arrière de la semelle du pied prothétique (3).

4. Procédé de fabrication d'une prothèse selon la revendication 1, selon lequel on moule en une matière thermoplastique ou en un matériau stratifié une ossature monobloc de la partie de jambe (2) et du pied prothétique (3) de la prothèse, autour d'un noyau (10) approprié, caractérisé en ce qu'après moulage, on découpe la semelle du pied prothétique pour extraire le noyau (10), et en ce que l'on ménage dans l'ossature moulée, au niveau du talon du pied prothétique et au-dessus de la semelle, une découpe (9) en forme de coin se prêtant à une flexion de l'arrière de la semelle par rapport à la partie de jambe.

5. Procédé selon la revendication 4, caractérisé en ce que, préalablement au moulage de la matière thermoplastique ou du matériau stratifié, on fixe sous le noyau (10) utilisé pour le thermoformage une semelle de renfort (8) en un matériau élastiquement déformable, de façon telle qu'elle soit rendue solidaire de ce matériau au cours de l'opération de thermoformage, et en ce que, après cette opération, on extrait le noyau (10) à travers une ouverture ménagée dans cette semelle et dans le matériau de thermoformage.

6. Procédé selon la revendication 5, caractérisé en ce que, après l'extraction du noyau (10), on introduit dans le pied prothétique une lame ressort métallique ou en un autre matériau déformable élastiquement arc-boutée et recourbée entre l'extrémité avant et l'extrémité arrière du pied prothétique.

## Patentansprüche

1. Prothese für Beinamputierte, bestehend aus einem warmgeformten Rahmen aus thermoplastischem Material oder Schichtwerkstoff, der mit einem Überzugsmaterial ummantelt ist und aus einer Hülse (1), einem Beinteil (2) und einem prothetischen Fuß (3) besteht, die jeweils miteinander verbunden sind, wobei der Rahmen des Beinteils (2) und des prothetischen Fußes (3) aus einem Stück bestehen, und zwar aufgrund eines einzigen Formvorganges; diese Prothese ist dadurch gekennzeichnet, daß der prothetische Fuß in Höhe der Sohle einen Ausschnitt (30), der zur Entnahme des Formkerns gedient hat und in Höhe der Ferse unterhalb der Sohle einen eckigen Ausschnitt (9) aufweist, der die Beugung der hinteren Sohle in bezug auf das Beinteil zuläßt.

2. Prothese nach Anspruch 1, dadurch gekennzeichnet, daß eine Sohle (8) aus einem elastisch verformbaren Material in den prothetischen Fuß (3) eingearbeitet ist.

3. Prothese nach Anspruch 2, dadurch gekennzeichnet, daß in den prothetischen Fuß (3) über der Sohle (8) aus elastisch verformbarem Material ein Federblatt aus Metall (16) eingearbeitet ist, dessen mittlerer Bereich ausgebaucht ist und dessen Enden an den vorderen und hinteren Enden der Sohle des prothetischen Fußes (3) anliegen.

4. Verfahren zur Herstellung eines Prothese nach Anspruch 1, nach dem ein aus thermoplastischem Material oder einem Schichtwerkstoff und aus einem Stück bestehender Rahmen des Beinteils (2) und prothetischen Fußes (3) der Prothese um einen geeigneten Formkern (10) geformt wird, dadurch gekennzeichnet, daß nach dem Formen die Sohle des prothetischen Fußes eingeschnitten wird, um den Formkern (10) zu entfernen, und dadurch, daß ein eckiger Einschnitt (9) in dem geformten Rahmen in Höhe der Ferse des prothetischen Fußes oberhalb der Sohle vorgesehen wird, der die Beugung der Sohle in bezug auf das Beinteil zuläßt.

5. Verfahren nach Anspruch 4, dadurch gekennzeichnet, daß vor dem Formen des thermoplastischen Materials oder des Schichtwerkstoffes unter dem zum Warmformen verwendeten Formkern (10) eine Verstärkungssohle (8) aus elastisch verformbarem Material befestigt wird, damit sich diese im Laufe des Warmformens mit diesem Material verbindet, und dadurch, daß nach diesem Arbeitsgang der Formkern (10) durch eine in dieser Sohle und in dem Warmformwerkstoff ausgeführten Öffnung entfernt wird.

6. Verfahren nach Anspruch 5, dadurch gekennzeichnet, daß nach dem Entfernen des Formkerns (10) ein Federblatt aus Metall oder einem anderen elastisch verformbaren Werkstoff zwischen den vorderen und hinteren Enden des prothetischen Fußes abgestützt und gebogen eingeführt wird.

## Claims

1. A prosthesis for leg amputees comprising a thermoformed framework of a thermoplastic material or a laminated material, sheathed in a covering material and comprising a socket (1), a leg part (2) and a prosthetic foot (3) adjoining in pairs, the framework of the leg part (2) and of the prosthetic foot (3) constituting an integral unit, originating from a single moulding operation, this prosthesis being characterized in that the prosthetic foot comprises, at the level of the sole, a cut-out (30) serving for extraction of the mould core and, at the level of the heel, above the sole, a cut-out (9) of angular shape lending itself to bending of the rear of the sole with respect to the leg part.

2. A prosthesis according to claim 1, characterized in that a sole (8) of elastically deformable material is incorporated in the prosthetic foot (3).

3. A prosthesis according to claim 2, characterized in that in the prosthetic foot (3), above the sole (8) of elastically deformable material, there is incorporated a metal leaf spring (16), bulging in its central part and resting at its ends against the front and rear ends of the sole of the prosthetic foot (3).

4. A process of manufacturing a prosthesis according to claim 1, according to which an integral framework of the leg part (2) and of the prosthetic foot (3) of the prosthesis is moulded from a thermoplastic material or from a laminated material about a suitable core (10), characterized in that, after moulding, the sole of the prosthetic foot is cut to extract the core (10), and in that there is formed in the moulded framework, at the level of the heel of the prosthetic foot and above the sole, a cut-out (9) of angular shape lending itself to bending of the rear of the sole with respect to the leg part.

5. A process according to claim 4, characterized in that, before the thermoplastic or laminated material is moulded, a reinforcing sole (8) of elastically deformable material is fixed under the core (10) used for thermoforming, in such a way that it is rendered integral with this material during the thermoforming operation, and in that, after this operation, the core (10) is extracted through an opening formed in this sole and in the thermoforming material.

6. A process according to claim 5, characterized in that, after extraction of the core (10), a leaf spring of metal or of another elastically deformable material is introduced into the prosthetic foot, said leaf spring being braced and curved between the front and rear ends of the prosthetic foot.
